Europäisches Patentamt

European Patent Office

Office européen des brevets

(19)

(11) Veröffentlichungsnummer: **0 242 633 B1**

(12) # EUROPÄISCHE PATENTSCHRIFT

(45) Veröffentlichungstag der Patentschrift: 27.03.91

(51) Int. Cl.⁵: **A61F 2/34**

(21) Anmeldenummer: 87104537.3

(22) Anmeldetag: 27.03.87

(54) **Aus einem inneren Pfannenkörper und einer Aussenschale bestehende, für eine zementfreie Verankerung geeignete Endoprothese für eine Hüftgelenkspfanne.**

(30) Priorität: 15.04.86 CH 1489/86

(43) Veröffentlichungstag der Anmeldung:
28.10.87 Patentblatt 87/44

(45) Bekanntmachung des Hinweises auf die
Patenterteilung:
27.03.91 Patentblatt 91/13

(84) Benannte Vertragsstaaten:
AT BE DE ES FR GB IT NL SE

(56) Entgegenhaltungen:
EP-A- 0 169 978
DE-A- 2 911 754
FR-A- 2 366 005

(73) Patentinhaber: **GEBRÜDER SULZER AKTIENGE-
SELLSCHAFT**
**Zürcherstrasse 9**
**CH-8401 Winterthur(CH)**

Patentinhaber: **Protek AG**
**Stadtbachstrasse 64**
**CH-3001 Bern(CH)**

(72) Erfinder: **Spotorno, Lorenzo, Dr.-med.**
**Ospedale Riunitit**
**I-17024 Finale Ligure(IT)**
Erfinder: **Frey, Otto**
**Wallrütistrasse 56**
**CH-8400 Winterthur(CH)**

## Beschreibung

Die Erfindung betrifft eine aus einem inneren Pfannenkörper und einer Aussenschale bestehende, für eine zementfreie Verankerung geeignete Endoprothese für eine Hüftgelenkspfanne, bei der der Pfannenkörper mit einem konischen Mantel und die Aussenschale mit einem, an den Mantel angepassten Hohlraum versehen und über eine Verriegelungsstruktur ineinander gehalten sind, wobei auf dem Umfang der aussen mindestens annähernd Halbkugelform aufweisenden Aussenschale eine Anzahl in Meridianrichtung verlaufender Schlitze verteilt sind, und wobei ferner im zum Aequator gelegenen Bereich der Aussenschalen-Oberfläche eine aus in mehreren peripheren Reihen angeordneten Vorsprüngen bestehende Struktur vorhanden ist.

Eine Prothese der vorstehend genannten Art ist bekannt aus der EP-A-O 169 978; für die Verankerung im Beckenknochen wird die Aussenschale dieser Prothese zunächst mit Hilfe eines Werkzeugs elastisch zusammengepresst und in Richtung ihrer Achse in eine operativ geschaffene Ausnehmung im Beckenknochen eingesetzt. Nach dem Entfernen des Werkzeugs wird der Pfannenkörper eingetrieben, durch den ein Aufspreizen der geschilderten Aussenschale erfolgt, wobei die Vorsprünge der Struktur in das die Wand der Ausnehmung bildende Knochengewebe eindringt.

Das Aufspreizen der Aussenschale stellt eine Drehung der einzelnen, durch die Schlitze getrennten "Lappen" des äquatornahen Bereichs der Schale um Achsen dar, die jeweils zwei benachbarte Schlitze an den Orten der grössten "Lappen-Einschnürung" verbinden. Die Lappen dringen bei dieser Drehung auf Kreisbögen in das Gewebe ein. Dabei hat sich bei den bisherigen Vorsprüngen, die im äquatornahen Bereich als in Radialrichtung stehende, zu ihrer Achse symmetrische Zotten ausgebildet sind, gezeigt, dass infolge von auf die Zotten wirkenden axialen Kraftkomponenten parallel zur Symmetrieachse der Prothese die Aussenschale wieder aus dem Knochen herausgedrückt wird, wobei die Spongiosa durch die polseitigen Flanken der Zotten praktisch nicht komprimiert wird, was die Haftung verringert.

Aufgabe der Erfindung ist es, durch die Form der Dorne zu erreichen, dass die auf die polseitige und die äquatorseitige Flanke wirkenden Axialkräfte mindestens annähernd gleich gross sind, und dass weiterhin die Spongiosa durch beide Flanken mindestens annähernd gleich stark verdichtet wird.

Diese Aufgabe wird mit der Erfindung dadurch gelöst, dass die polwärts weisenden Flanken der Vorsprünge in Ebenen senkrecht zur Symmetrieachse der Prothese verlaufen, dass ferner ihre zum Aequator zeigenden Flanken mit diesen Ebenen Winkel zwischen 15° und 45° einschliessen, und dass schliesslich die Konen von Pfannenkörper und Aussenschale über ein Gewinde miteinander verbunden sind.

Infolge der beanspruchten Form der Vorsprünge liegt die Spitze eines Vorsprungs mindestens annähernd in der Mitte des durch die Flankenform und die Dicke des Vorsprungs an seinem Fusse gegebenen "Verdichtungsbandes" der Spongiosa. Die auf die dem Aequator zugewandte und auf die gegenüberliegende Flanke wirkenden Axialkräfte kompensieren sich mindestens annähernd, so dass ein Herausdrücken der Aussenschale aus dem Knochen durch eine stark überwiegende Axialkraft in Richtung auf den Aequator vermieden ist.

Um den für die einzelnen peripheren Reihen von Vorsprüngen unterschiedlichen "Spitzenradien" Rechnung zu tragen, ist es zweckmässig, wenn die Flankenwinkel bei einzelnen peripheren Reihen unterschiedlich sind, wobei polnähere Reihen relativ zu polfernen Reihen grössere Flankenwinkel haben. Um die Fertigung der üblicherweise aus einem der in der Implantat-Technik verwendeten Metalle bestehenden Aussenschale zu vereinfachen, ist es zweckmässig, wenn die Spitzen der Vorsprünge der einzelnen Reihen auf gemeinsamen Mantellinien angeordnet sind und/oder wenn die Zwischenräume zwischen den Vorsprüngen in Umfangsrichtung durch konkave Kreislinien begrenzt sind. Auf diese Weise lassen sich die Vorsprünge durch einfaches Fräsen entlang von Mantellinien der Aussenform der Schale herstellen.

Im folgenden wird die Erfindung anhand eines Ausführungsbeispiels im Zusammenhang mit der Zeichnung näher erläutert.

Fig. 1    ist eine Aufsicht in Richtung der Symmetrieachse der Prothese auf eine Aussenschale;

Fig. 2    gibt den Schnitt II-II von Fig. 1 wieder;

Fig. 3    zeigt einen der Fig. 2 entsprechenden Schnitt durch einen Pfannenkörper;

Fig. 4    schliesslich stellt in vergrössertem MassStab das Detail D von Fig. 2 dar.

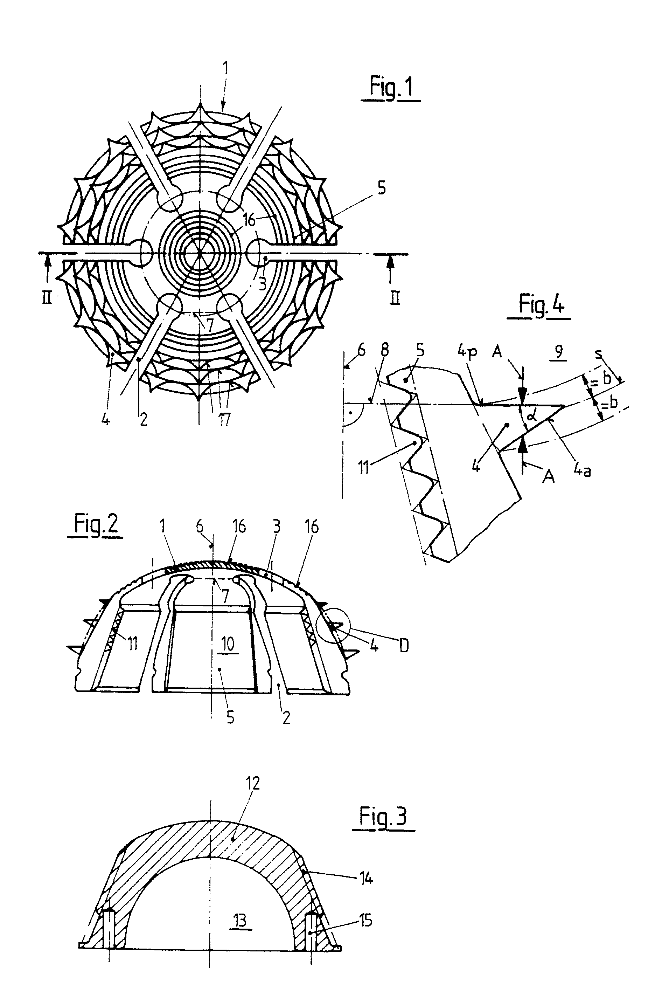

Die Aussenschale 1 (Fig. 1) bildet mindestens annähernd eine Haublkugelschale, in der gleichmässig über den Umfang verteilt Meridianschlitze 2 vorgesehen sind, durch die der äquatornahe Bereich der Aussenschale 1 in einzelne Lappen 5 unterteilt wird; die Symmetrieachse der Schale bzw. Prothese ist mit 6 bezeichnet (Fig. 2). Die Schlitze 2 enden in kreisförmigen Erweiterungen 3, zwischen denen dadurch für elastische Verformungen bevorzugte Bereiche "maximaler Lappen-Einschnürungen" entstehen, in denen die "Drehachsen" 7 für das Aufspreizen der Lappen 5 liegen. Die Mittelpunkte der kreisförmigen Erweiterungen 3 sind etwa auf 65° geographischer Breite angeordnet.

Aussen ist auf der Aussenschale 1 eine Vielzahl von Vorsprüngen 4 vorgesehen, die aus der äusseren Oberfläche der Aussenschale 1 dornartig hervorstehen. Erfindungsgemäss sind die Vorsprünge 4 so gestaltet, dass ihre polwärts gerichteten Flanken 4p (Fig. 4) in Ebenen 8 senkrecht zur Achse 6 verlaufen, während die dem Aequator zugewandten Flanken 4a (Fig. 4) mit den Ebenen 8 Winkel α einschliessen.

Wie in Fig. 4 schematisch angedeutet, dringt die Spitze eines Dorns oder Vorsprunges 4 in das Knochengewebe 9 beim Aufspreizen der Aussenschale 1 derart ein, dass das Gewebe 9 von beiden Flanken 4p und 4a mindestens annähernd in gleichem Masse verdrängt und verdichtet wird. Dadurch werden der Verdichtungsbereich b und damit die axialen Kraftkomponenten zu beiden Seiten der Spur s der Dornspitze mindestens annähernd gleich, so dass sich die axialen Kraftkomponenten A gegenseitig mindestens nahezu aufheben.

Aus Fertigungsgründen sind die Spitzen der Dorne 4 der einzelnen peripheren Reihen 17 (Fig. 1) in Mantellinien der Aussenschale 1 übereinander angeordnet; durch die bereits erwähnte Herstellung mit Hilfe eines Fräsers bedingt, sind die Zwischenräume in Umfangsrichtung von konkaven Kreislinien bzw. Ausschnitten aus Zylindermantelflächen begrenzt (Fig. 1).

Im Polbereich ist die Aussenschale 1 zur Vergrösserung der "Anwachsoberfläche" mit in Umfangsrichtung verlaufenden Rillen 16 versehen.

Der Hohlraum 10 der Aussenschale 1 hat im wesentlichen die Form eines Kegelstrumpfes, der einseitig durch eine Bohrung abgeschlossen ist. Sein seitlicher Mantel ist mit einem Gewinde 11 (Fig. 2) versehen. Das Gegenstück zum Hohlraum 10 bildet der Pfannenkörper 12, in den die eigentliche Pfannenschale 13 eingearbeitet ist. Er besitzt ein Gegengewinde 14, mit dem er in das Gewinde 11 des Hohlraums 10 eingeschraubt wird; Bohrungen 15 im Rand des Pfannenkörpers 12 dienen dem Einsatz eines entsprechenden Einschraubwerkzeuges.

Beim Einschrauben übt der Pfannenkörper 12 auf die Aussenschale 1 eine radial gerichtete Aufweitkraft aus, durch die Lappen 5 in den Beckenknochen eingepresst werden, wobei die Dorne 4 in der beschriebenen Weise in das Gewebe 9 eindringen und sich dort verankern.

## Ansprüche

1. Aus einem inneren Pfannenkörper (12) und einer Aussenschale (1) bestehende, für eine zementfreie Verankerung geeignete Endoprothese für eine Hüftgelenkspfanne, bei der der Pfannenkörper (12) mit einem konischen Mantel und die Aussenschale (1) mit einem, an den Mantel angepassten Hohlraum versehen und über eine Verriegelungsstruktur ineinander gehalten sind, wobei auf dem Umfang der aussen mindestens annähernd Halbkugelform aufweisenden Aussenschale eine Anzahl in Meridianrichtung verlaufender Schlitze (2) verteilt sind, und wobei ferner im zum Aequator gelegenen Bereich der Aussenschalen-Oberfläche eine aus in mehreren peripheren Reihen angeordneten Vorsprüngen (4) bestehende Struktur vorhanden ist, dadurch gekennzeichnet, dass die polwärts weisenden Flanken (4p) der Vorsprünge (4) in Ebenen (8) senkrecht zur Symmetrieachse (6) der Prothese verlaufen, dass ferner ihre zum Aequator zeigenden Flanken (4a) mit diesen Ebenen (8) Winkel (α) zwischen 15° und 45° einschliessen, und dass schliesslich die Konen von Pfannenkörper und Aussenschale über ein Gewinde (11,14) miteinander verbunden sind.

2. Endoprothese nach Anspruch 1, dadurch gekennzeichnet, dass die Spitzen der Vorsprünge (4) der einzelnen Reihen (17) auf gemeinsamen Mantellinien angeordnet sind.

3. Endoprothese nach Anspruch 1 oder 2, dadurch gekennzeichnet, dass die Zwischenräume zwischen den Vorsprüngen (4) in Umfangsrichtung durch konkave Kreislinien begrenzt sind.

4. Endoprothese nach Anspruch 1, dadurch gekennzeichnet, dass die Flankenwinkel (α) bei einzelnen peripheren Reihen (17) unterschiedlich sind, wobei polnähere Reihen (17) relativ zu polfernen Reihen (17) grössere Flankenwinkel (α) haben.

## Claims

1. A non-cement-fixing acetabular endoprosthesis, comprising an inner cup (12) and an outer shell (1), the cup (12) having a conical envelope surface and the outer shell (1) having a hollow interior adapted to such surface, the cup (12) and the outer shell (1) being retained in one another by way of a locking structure, a number of slots (2) which extend in the meridian direction being distributed over the periphery of the outer shell, the same being externally at least substantially hemispherical, while a structure which comprises projections (4) disposed in a number of peripheral rows is present in that zone of the outer shell surface which is near the equator, characterised in that those flanks (4p) of the projections (4) which extend towards the pole extend in planes (8) perpendicularly to the axis (6) of symmetry of the prosthesis, their flanks (4a) which extend towards the equator include angles (α) of from 15° to 45° with the planes (8), and the cones of the cup (12) and outer shell (1) are interconnected by way of screwthreading (11, 14).

2. An endoprosthesis according to claim 1, characterised in that the apexes of the projections (4) of the individual rows (17) are disposed on common generatrices.

3. An endoprosthesis according to claim 1 or 2, characterised in that the gaps between the projections (4) are bounded in the peripheral direction by concave circle lines.

4. An endoprosthesis according to claim 1, characterised in that the flank angles (α) differ in individual peripheral rows (17), rows (17) which are relatively near the pole having greater flank angles (α) than rows (17) which are relatively far from the pole.

**Revendications**

1. Endoprothèse pour la cavité d'articulation de la hanche se composant d'un cotyle interne (12) et d'une coguille externe (1) et convenant à un encastrement sans ciment, prothèse dont le cotyle (12) comporte une enveloppe conigue et la coquille externe (1) comporte une cavité épousant ladite enveloppe, ledit cotyle et ladite coquille étant maintenus l'un dans l'autre par une structure de verrouillage, plusieurs rentes (2) orientées dans la direction de méridiens étant réparties à la circonférence de la coquille externe qui a au moins approximativement une forme hémisphérigue et, par ailleurs, une structure se composant de saillies (4) disposées en plusieurs rangées périphériques se trouvant dans la région de la surface de la coquille externe qui est proche de l'équateur, caractérisée en ce que les flancs (4p) des saillies (4) qui sont tournés vers le pôle sont situés dans des plans (8) perpendiculaires à l'axe de symétrie (6) de la prothèse, en ce que, par ailleurs, les flancs (4a) tournés vers l'équateur inscrivent avec ces plans (8) un angle (α) compris entre 15 et 45° et, finalement, en ce que les cônes du corps du cotyle et de la coquille externe sont reliés par filetage (11, 14).

2. Endoprothèse selon la revendication 1, caractérisée en ce que les pointes des saillies (4) des différentes rangées (17) sont disposées le long de génératrices communes.

3. Endoprothèse selon la revendication 1 ou 2, caractérisée en ce que les intervalles séparant les saillies (4) dans la direction de la circonférence sont délimités par des arcs de cercle concaves.

4. Endoprothèse selon la revendication 1, caractérisée en ce que les angles entre flancs (α) diffèrent d'une rangée périphérique à l'autre (17), l'angle (α) entre flancs des rangées (17) proches du pôle étant plus grand que celui des rangées (17) qui en sont plus éloignées.

Fig. 1

Fig. 4

Fig. 2

Fig. 3

5